Europäisches Patentamt

European Patent Office

Office européen des brevets

(19)

(11) Publication number: **0 210 722**
**B1**

# EUROPEAN PATENT SPECIFICATION

(12)

(45) Date of publication of the patent specification:
**17.01.90**

(51) Int. Cl.⁴: **A61B 3/10, G01M 11/02**

(21) Application number: **86303669.5**

(22) Date of filing: **14.05.86**

(54) **Apparatus for measuring the refractive power or radius of curvature of an optical system.**

(30) Priority: **05.06.85 JP 121938/85**

(43) Date of publication of application:
**04.02.87 Bulletin 87/6**

(45) Publication of the grant of the patent:
**17.01.90 Bulletin 90/3**

(84) Designated Contracting States:
**DE FR GB IT**

(56) References cited:
**DE-A- 3 118 560**
**US-A- 3 136 839**
**US-A- 3 715 166**
**US-A- 4 190 332**
**US-A- 4 353 625**
**US-A- 4 526 451**
**US-A- 4 572 628**

**Patent abstracts of Japan, unexamined applications, P
field, vol. 7, no. 10 (P-168), January 14, 1983**

(73) Proprietor: **Sun High-Tech Kabushiki Kaisha, 19-20,
Morisaki 2-Chome, Yokosuka-Shi Kanagawa-Ken(JP)**

(72) Inventor: **Nohda, Masao, 41-18, Oyabe 3-Chome,
Yokosuka-Shi Kanagawa-Ken(JP)**

(74) Representative: **Cockbain, Julian Roderick Michaelson
et al, Frank B. Dehn & Co. European Patent Attorneys
Imperial House 15-19, Kingsway, London
WC2B 6UZ(GB)**

## Description

This invention relates to an apparatus for measuring the refractive power or radius of curvature of an optical system, and particularly to such an apparatus for measuring the eye's refractive power, to an automatic lens meter or to a radius of curvature measuring apparatus.

For measuring the eye's refractive power there are fundamentally three kinds of measuring systems: a coinciding system, an image forming system and a shadow inspecting system. The present invention insofar as it relates to an apparatus for measuring the eye's refractive power utilizes the shadow inspecting system.

First of all, the prior art shall be explained with reference to an example of an apparatus for measuring the eye's refractive power.

On the conventional automatic eye refractive power measuring apparatus, there have been made various improvements to simplify the mechanism, to facilitate production and adjustment, to reduce measuring time and to increase measuring precision. Thus, an automatic eye refractive power measuring apparatus is shown in Japanese Unexamined Patent Application Publication No. 160538/1980.

However, in this automatic eye refractive power measuring apparatus, there have been defects in that, as an image rotating prism is required to rotate beams, it is difficult in the manufacture and adjustment to rotate the image rotating prism around the optical axis as a center without errors, and light amount will attenuate due to the reflection of the image rotating prism and the measuring time can not be reduced so much.

Therefore, in order to eliminate such defects, an automatic eye refractive power measuring apparatus shown in the Japanese Unexamined Patent Application Publication No. 165735/1982 (US-A 4 526 451) has been provided.

In this automatic eye refractive power measuring apparatus, the eye to be inspected is periodically scanned alternatively in two known directions with slit-shaped illuminating beams, the slits being at the same angle of inclination to the scanning direction, the reflected light is received by two pairs of photoelectric converting elements and the phase difference between the output signals of the photoelectric converting elements forming the respective pairs is measured to determine the refractive power of the eye under investigation.

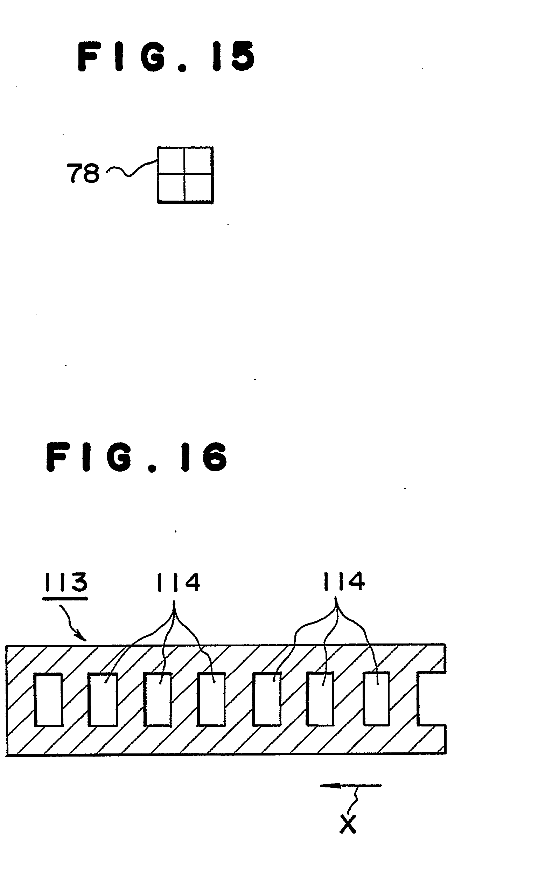

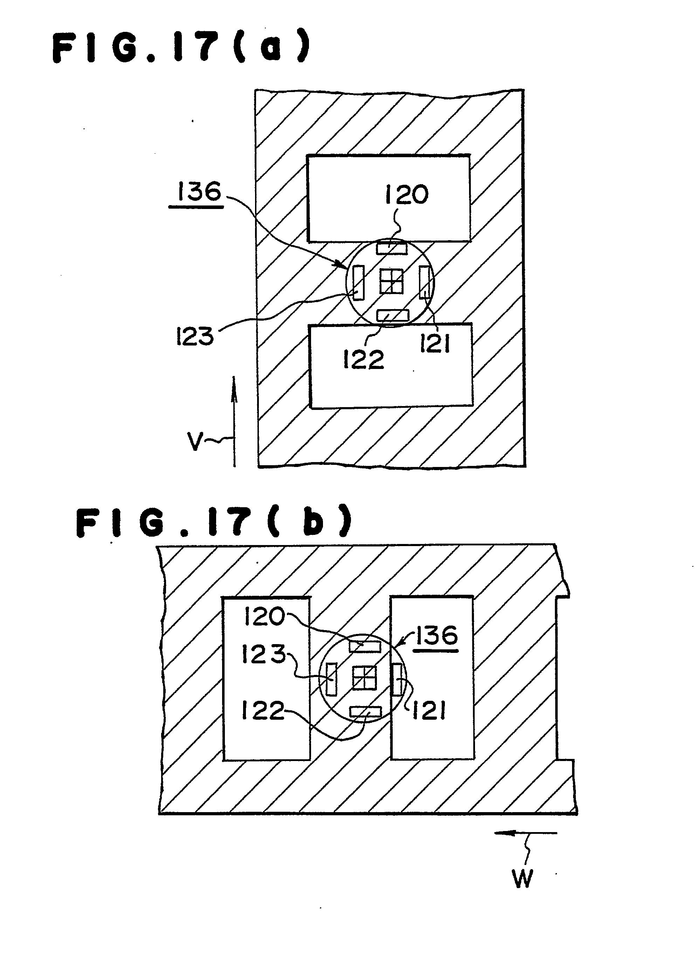

For a shadow projecting device making the above mentioned slit-shaped illuminating beams scan alternatively in two known directions, as shown in Figs. 16 and 17 hereto, there are used a light source, a prism and a rotary light beam chopper 113 provided with slit apertures 114 formed to be all at the same angle of inclination to the rotating direction of rotation X. Fig. 16 is a developed view of the rotary chopper 113.

Now, if it is assumed that there is no astigmatism in the eye to be inspected, and as in Fig. 17(a) where the above mentioned slit-shaped illuminating beams are made to scan the eye in one direction or as in Fig. 17(b) where the slit-shaped illuminating beams are made to scan in the other direction, the light reflected from the eye of the slit-shaped illuminating beams will run respectively in the direction indicated by the arrow V or W through two pairs of photoelectric converting elements 120, 122 and 121, 123 on the light receiving surface of a photoelectric converter 136.

If it is assumed that there is an astigmatism in the eye being inspected, the above mentioned reflected light will deviate in response to an angle (which shall be hereinafter called cylinder axis angle) $\theta$ made by the main warp line of the astigmatism with one scanning direction of the above-mentioned slit shaped illuminating beams in the plane of the eye being inspected as compared with the illustration in Figs. 17(a) and 17(b).

Therefore, if S represents a spherical refractive power and C represents a cylindrical refractive power, in the case where the above mentioned slit-shaped illuminating beams are made to scan in the direction corresponding to Fig. 17(a), the value $D_1$ obtained from the phase difference of the output signals of the photoelectric converting elements 120 and 122 will be

$$D_1 = S + C \sin^2 \theta \qquad (1)$$

and the value $D_2$ obtained from the phase difference of the output signals of the photoelectric converting elements 121 and 123 will be

$$D_2 = \frac{C}{2} \sin 2\theta \qquad (2)$$

Also, in the case where the above mentioned slit-shaped illuminating beams are made to scan in the direction corresponding to Fig. 17(b), the value $D_3$ obtained from the phase difference of the output signals of the photoelectric converting elements 120 and 122 will be

$$D_3 = -\frac{C}{2} \sin 2 \theta \quad , \quad (3)$$

and the value $D_4$ obtained from the phase difference of the output signals of the photoelectric converting elements 121 and 123 will be

$$D_4 = S + C \cos^2 \theta \quad (4)$$

Therefore, from the above equations (1) to (4), the spherical refractive power S, cylindrical refractive power C and column axis angle $\theta$, that is the refractive power, can be determined by operation.

The derivation of equations (1) to (4) is described in Japanese Unexamined Patent Application Publication No. 160538/1980 to which the reader is referred.

However, in the automatic eye refractive power measuring apparatus shown in the above mentioned Japanese Unexamined Patent Application Publication No. 165735/1982, as described above, the slit-shaped illuminating beams must be made to scan alternatively in two known directions, therefore, instead of using an image rotating prism, a prism which is not required in Japanese Unexamined Patent Application Publication No. 160538/1980 is required, the effectiveness to the light amount is not so high, two light sources are required instead of one light source required in Japanese Unexamined Patent Application Publication No. 160538/1980 and there is provided a new defect in the manufacture in that the beams and light amounts of the two light sources must be made to coincide respectively with each other.

By the way, as the eye is also an optical system, it is apparent that the above described argument is not limited to the automatic eye refractive power measuring apparatus but can be also established for such refractive power measuring apparatus of other optical systems, such as a lens meter or for a radius of curvature measuring apparatus.

That is to say, the same defect as is described above is produced also, for example, in a lens meter (e.g. that described in Japanese Unexamined Patent Application Publication No. 168137/1982 to which the above mentioned Japanese Unexamined Patent Application Publication No. 165735/1982 is applied and an automatic radius of curvature measuring apparatus (e.g. that described in Japanese Unexamined Patent Application Publication No. 197405/1982).

The present invention has as an object the provision of an apparatus for measuring the refractive power or radius of curvature of an optical system in which the defects discussed above are reduced or eliminated.

Therefore, according to one aspect of the present invention, there is provided an apparatus for measuring the refractive power of an optical system or the radius of curvature of a lens of an optical system, said apparatus comprising:

means for emitting light to impinge on said optical system;

means for directing said light from said optical system onto at least two pairs of photoelectric converting elements;

means for scanning said light over said optical system and/or said converting elements, said means defining beams of elongate cross-section; and

determining means for determining said refractive power or radius of curvature on the basis of the phase differences between the output signals of said pairs of converting elements,

characterized in that said means for scanning is arranged to define said beams with the elongate cross-sections thereof inclined at at least two angles of inclination relative to the direction of scanning (X) and in that said apparatus comprises means for providing to said determining means a signal identifying said angle of inclination of the light beam scanning said optical system and/or said converting elements.

In one embodiment, the apparatus of the invention is an apparatus for measuring the refractive power of an optical system comprising: means for scanning an optical system under investigation with light beams of elongate cross section; means for detecting light from said beams reflected from said system, said means for detecting comprising at least two pairs of photoelectric converting elements; and determining means for determining said refractive power on the basis of the phase difference between the output signals of the photoelectric converting elements in the said pairs, wherein said means for scanning is so arranged as to scan said system with light beams whose elongate cross sections are inclined at two or more angles of inclination to the direction of scanning and wherein said apparatus comprises means for providing to said determining means a signal identifying the said angle of inclination of the light beam scanning said optical system.

According to the present invention, with the above mentioned embodiment, as the angle of inclination of the slit-shaped illuminating beams to the scanning direction of the inspected optical system by the slit-shaped illuminating beams is made two or more different known angles, simply to scan the optical system in one direction with the slit-shaped illuminating beams will be substantially equivalent to the prior art procedure involving alternatively scanning the optical system in two known directions with slit-shaped illuminating beams all lying at the same angle of inclination to the scanning direction.

Therefore, with the apparatus of the present invention, it is not necessary to make the slit-shaped illuminating beams scan alternatively in two known directions, no prism is required, only one light source will do, further it is not necessary to use an image rotating prism; therefore, manufacture of the apparatus is simplified, measuring time is reduced and high precision measurements are possible.

Preferred embodiments of the apparatus of the invention will now be described by way of example and with reference to the accompanying drawings, in which:-

Fig. 1 is a schematic plan view of one embodiment of the apparatus of the present invention;

Fig. 2 is a plan view of a diaphragm in the apparatus of Fig. 1;

Fig. 3 is a view of the apparatus of Fig. 1 in the direction indicated by the arrows A-A';

Fig. 4 is a circuit diagram for the apparatus of Fig. 1;

Fig. 5 is a developed view of a rotary light beam chopper for the apparatus of Fig. 1;

Figs. 6(a) and 6(b) are explanatory views showing scanning by the apparatus of Fig. 1 by the reflected light of the slit shaped beams;

Figs. 7(a) and 7(b) are further explanatory views showing another embodiment and corresponding respectively to Figs. 6(a) and 6(b);

Fig. 8 is a further developed view showing another embodiment of a rotary light beam chopper and corresponding to Fig. 5;

Fig. 9 is a further developed view showing another embodiment of a rotary light beam chopper and corresponding to Fig. 5;

Figs. 10(a), 10(b) and 10(c) are further explanatory views showing another embodiment and corresponding to Figs. 6(a) and 6(b);

Fig. 11 is a schematic plan view of a further embodiment of the apparatus of the invention;

Fig. 12 is a plan view of a diaphragm in the apparatus of Fig. 11;

Fig. 13 is a view of the apparatus of Fig. 11 in the direction indicated by the arrows B-B';

Fig. 14 is a schematic plan view of a further embodiment of the apparatus of the invention;

Fig. 15 is a view of the apparatus of Fig. 14 in the direction indicated by the arrows C-C';

Fig. 16 is a developed view showing a prior art rotary light beam chopper and corresponding to Fig. 5; and

Figs. 17(a) and 17(b) are explanatory views showing prior art apparatus and corresponding respectively to Figs. 6(a) and 6(b).

The present invention shall now be described in detail with reference to an embodiment utilized as an automatic eye refractive power measuring apparatus.

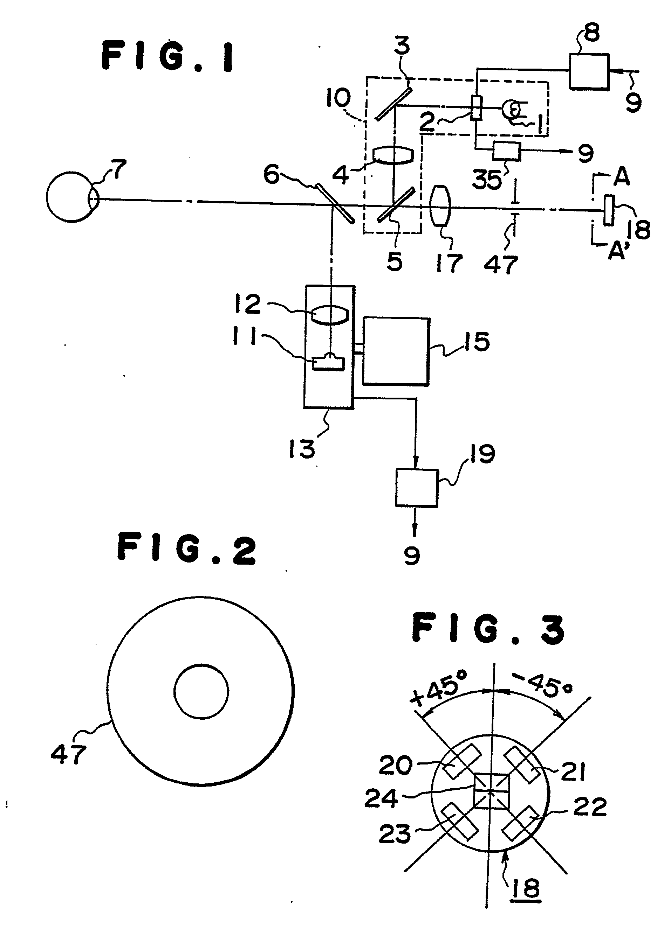

Referring to Figure 1, there is shown an embodiment of an automatic eye refractive power measuring apparatus.

A light source 1 is arranged to illuminate a visual target 2. Light beams from the visual target 2 are reflected by a reflector 3 and are brought into the form of substantially parallel beams by a collimator lens 4. These beams are reflected by a first light path divider 5, before passing through a second light path divider 6 and inpinging on the eye 7 which is being inspected and which is fixedly sighted on the visual target 2. Here, the visual target 2 is movable in the optical axis direction by a driving means 8. As described later, the driving means 8 is controlled by the output of an operating circuit 9 so that the visual target 2 may be movable to a position in which the eye 7 can fixedly sight the visual target as unadjusted to form a so-called automatic fog device. In the above, a visual target optical system 10 is formed of the light source 1, visual target 2, reflector 3, collimator lens 4 and first light path divider 5. This visual target optical system 10 is used to make the eye 7 being inspected fixedly sight the visual target 2 during the measurement and to reduce the instrument myopia of the eye caused during the measurement.

Further, the reference numeral 11 represents a light source such as, for example, an infrared LED (light emitting diode), 12 represents a condenser lens for forming an image of the light source 11 on the cornea of the eye 7 being inspected, 13 represents a rotary chopper provided with slit-shaped apertures 14a and 14b as detailed later and 15 represents a motor for rotating this rotary chopper at a fixed velocity in a fixed direction.

Therefore, the beam emitted from the light source 11 passes through the condenser lens 12, is then chopped by the rotary chopper 13 into slit-shaped illuminating beams, is further reflected by the second light path divider 6, is condensed on the cornea of the eye 7 and is projected on the bottom of the eye. That is to say, the slit-shaped illuminating beams scan the eye 7 in one direction.

Further, the reference numeral 18 represents a photoelectric converter, 17 represents a lens for conjugating this photoelectric converter 18 and the cornea of the eye 7 with each other and 47 represents a diaphragm having a circular aperture with the optical axis as a center (its shape as seen in the optical axis directed being shown in Fig. 2).

Therefore, the slit-shaped illuminating beams projected on the bottom of the above mentioned eye 7 being inspected are reflected by the eye bottom, pass through the first light path divider 5 and second light path divider 6, are condensed by the lens 17, pass through the diaphragm 47 and illuminate the light receiving surface of the photoelectric converter 18.

In the present invention, the angle of inclination of the above mentioned slit-shaped illuminating beams to the scanning direction of the eye 7 is made two or more different known angles.

4

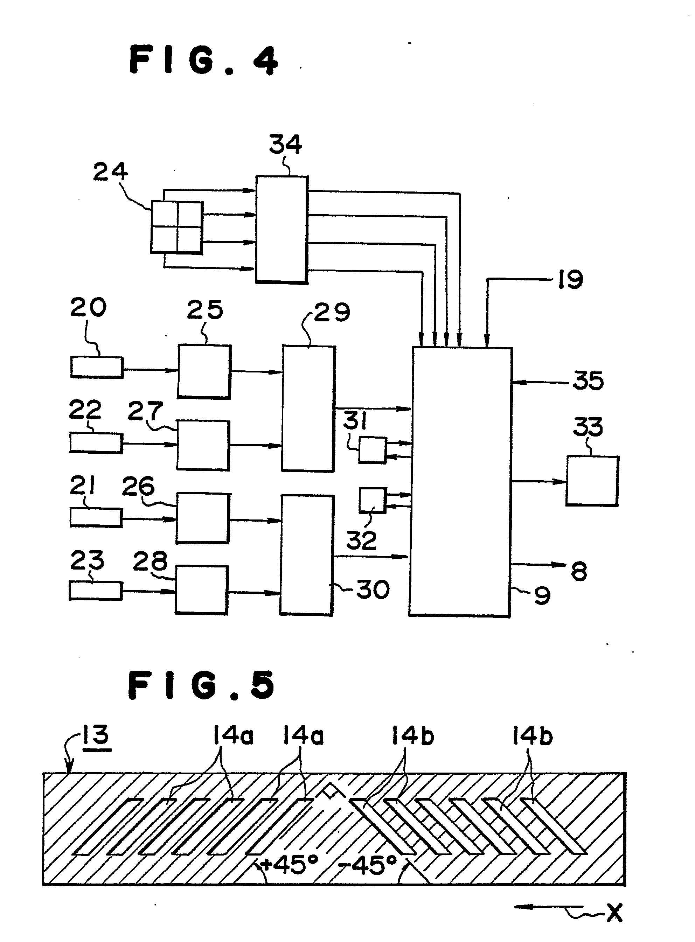

In the case of the illustrated embodiment, as shown in Fig. 5 showing the developed view of the rotary chopper 13, the angle of inclination of the first series of slit-shaped apertures 14a to the rotating direction X of the rotary chopper 13 is made +45 degrees, the angle of inclination of the second series of slit-shaped apertues 14b is made -45 degrees and thus the angle of inclination of the above mentioned slit-shaped illuminating beams to the scanning direction of the eye 7 being inspected is made two or more different known angles.

Further, in the present invention, there is provided an output means 19 putting out a discriminating signal showing with what angle of inclination of the slit-shaped illuminating beams the eye 7 is being scanned (which shall merely be called a discriminating signal hereinafter).

In the case of the illustrated embodiment, this output means 19 is, for example, a sensor for detecting the angle of rotation of the rotary chopper 13 corresponding to the position of the slit-shaped aperture 14a or 14b.

On the light receiving surface of the photoelectric converter 18, there are provided four photoeletric converting elements 20, 21, 22 and 23 around the optical axis and a four-divided photoelectric converting element 23 with the optical axis as a center of the division such as in Fig. 3 as already known (see the above mentioned Japanese Unexamined Patent Application Publication No. 165735/1982).

A pair of photoelectric converting elements 20 and 22 are arranged symmetrically with each other with respect to the optical axis in one measuring warp line direction, that is, on the line at +45 degrees with the line intersecting rectangularly with the optical axis within the paper surface of Fig. 1 and another pair of photoelectric converting elements 21 and 23 are arranged symmetrically with each other with respect to the optical axis in the measuring warp line direction intersecting rectangularly with the above mentioned warp line direction, that is, on the line at -45 degrees with the line intersecting rectangularly with the optical axis within the paper surface of Fig. 1.

The four-divided photoelectric converting element 24 receives the reflected light from the eye 7 for alignment. When the eye 7 being inspected and the measuring apparatus of the embodiment are well aligned with each other, the reflected image will be equally incident upon the respective elements of the four-divided photoelectric converting element and the outputs of the respective elements will be equal but, when they are not well aligned, the outputs of the respective elements will not be equal and the direction of the deviation will be determinable from from the difference in the outputs of the respective elements.

As shown in Fig. 4, the photoelectric converting elements 20, 21, 22 and 23 are connected respectively to waveform shaping circuits 25, 26, 27 and 28. The pair of waveform shaping circuits 25 and 27 shaping the waveforms of the output signals of the pair of photoelectric converting elements 20 and 22 are connected to a first phase difference measuring circuit 29 and the pair of waveform shaping circuits 26 and 28 shaping the waveforms of the output signals of the pair of photoelectric converting elements 21 and 23 are connected to a second phase difference measuring circuit 30. The first phase difference measuring circuit 29 and the second phase difference measuring circuit 30 are connected to an operating circuit 9. To the operating circuit 9 is further connected the above mentioned output means 19 so that the discriminating signal may be fed in. The operating circuit 9 memorizes respectively in memory circuits 31 and 32 the outputs of the first phase difference measuring circuit 29 and second phase difference measuring circuit 30 when the slit-shaped illuminating beams based on the first slit-shaped apertures 14a are scanning the eye 7 and the outputs of the first phase difference measuring circuit 29 and second phase difference measuring circuit 30 when the slit-shaped illuminating beams based on the second slit-shaped apertures 14b are scanning the eye 7, then operates as described later between the memorized values, derives the cylinder axis angle θ, spherical refractive power S and cylindrical refractive power C and indicates the results using an indicating device 33.

On the other hand, the outputs of the respective elements of the four-divided photoelectric converting element 24 are converted to digital signals by an A-D converter 34 and are then inputted to the operating circuit 9. The operating circuit 9 compares the sizes of the signals from the respective elements with the maximum value and inputs to the indicating device 33 a signal indicating the direction intersecting rectangularly with the optical axis and movement in the optical axis direction for alignment. The operating circuit 9 further receives the output of a position detecting means 35 for detecting the position on the optical axis of the visual target 2 and sends a signal to the driving means 8 so as to move the visual target 2 in turn by a fixed step in the direction of reducing the operated spherical refractive power S and cylindrical refractive power C. That is to say, such an automatic fogging device as is disclosed in U.S. Patent No. 4190332 is formed of the visual target 2, position detecting means 35, operating circuit 9 and driving means 8.

The operating of an embodiment of the apparatus of the present invention as an automatic apparatus for measuring the eye's refractive power will now be explained.

The inspector aligns the eye 7 under inspection and the measuring apparatus with each other while seeing the indication of the indicating means 33. After the completion of the alignment, when a measuring switch (not illustrated) is switched on, the measurement will begin.

When the slit-shaped illuminating beams based on the first slit-shaped apertures 14a are scanning the eye 7, the operating circuit 9 will discriminate the condition and will memorize in the memory circuit 31 the phase difference (the average value of many measurements being generally used) obtained from the phase difference measuring circuits 29 and 30. Further, when the slit-shaped illuminating beams based on the second slit-shaped apertures 14b are scanning the eye 7, the operating circuit 9 will discriminate the condition and will memorize in the memory circuit 32 the phase difference obtained from the phase difference measuring circuits 29 and 30. When the above mentioned memorization is completed, the operating circuit 9 will read the memories out of the memory circuit 31 and 32 and will derive the refractive power, that is, the cylinder axis angle θ, spherical refractive power S and cylindrical refractive power C of the eye being inspected. Then, the operating circuit 9 will input a signal to the driving means 8 for the visual target 2 so as to move the visual target 2 in the direction of relaxing the eye 7 on the basis of this refractive power, that is to say, the automatic fogging means will operate on the basis of the refractive power. The above described operation is repeated for each step until the spherical refractive power S no longer varies even if the visual target 2 is further varied, the operating circuit 9 will then indicate the refractive power in the indicating means.

The reason why the refractive power can be determined by the operation of the operating circuit shall be described in the following.

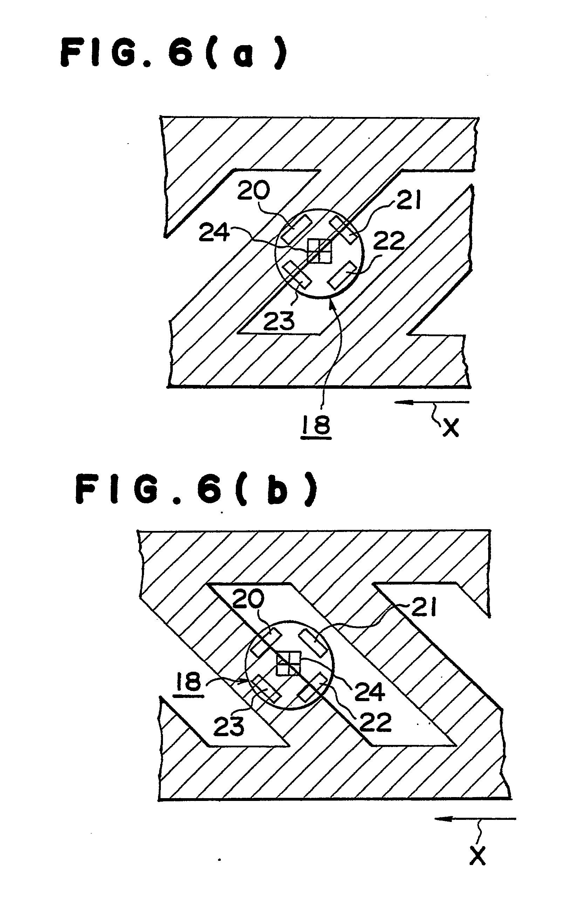

If there is no astigmatism in the eye 7 being inspected, when the slit-shaped illuminating beams based on the first slit-shaped apertures 14a are scanning the eye 7 being inspected, the reflected light will run on the light receiving surface of the photoelectric converter 18 in the direction indicated by the arrow X as in Fig. 6(a) and, when the slit-shaped illuminating beams based on the second slit-shaped apertures 14b are scanning the eye 7, the reflected lights will run in the direction indicated by the arrow X in Fig. 6(b). The condition of Fig. 6(a) will be substantially identical with the condition of Fig. 17(a) explained hereinbefore in the prior art discussion and the condition of Fig. 6(b) will be substantially identical with the condition of Fig. 17(b) explained hereinbefore in the prior art discussion.

Therefore, if where the slit-shaped illuminating beams based on the first slit-shaped apertures 14a scan the eye 7 being inspected, the value obtained from the phase difference of the output signals of the photoelectric converting elements 20 and 22 is represented by $D'_1$ and the value obtained from the phase difference of the output signals of the photoelectric converting elements 21 and 23 is represented by $D_2'$ and, where the slit-shaped illuminating beams based on the second slit-shaped apertures 14b scan the eye 7, the value obtained from the phase difference of the output signals of the photoelectric converting elements 20 and 22 is represented by $D_3'$ and the value obtained from the phase difference of the output signals of the photoelectric convering elements 21 and 23 is represented by $D_4'$, then

$$D_1' = S + C \cos^2 \theta \qquad (5)$$

$$D_2' = \frac{C}{2} \sin 2\theta \qquad (6)$$

$$D_3' = -\frac{C}{2} \sin 2\theta \qquad (7)$$

$$D_4' = S + C \sin^2 \theta \qquad (8)$$

Therefore, as there are three unknowns (C, S and θ) and three measured values, ($D_1'$, $D_2'$ (= $-D_3'$) and $D_4'$) then the operating circuit 9 may be set to determine the cylinder axis angle θ, the spherical refractive power S and the cylindrical refractive power C on the basis of equations (5), (6) (or (7)) and (8).

However, if such operation as is described above is made by the operating circuit 9, it will take time for the operation and the constants between the outputs of the phase difference measuring circuits 29 and 30 and the values $D_1'$, $D_2'$, $D_3'$ and $D_4'$ will vary depending on the arrangement of the optical system. Therefore, an actual apparatus in practice may be calibrated against an imitation eye of known refractive power; measuremets may be made by using the imitation eye the outputs of the phase difference measuring circuits 29 and 30 for the imitation eye being memorized by the operating circuit 9 as corresponding to the above described known refractive power to eliminate the above described disadvantages. As there are three unknowns (C, S and θ) and three measured data values (actually there are four data but two of them have axes intersecting rectangularly with each other and are different only in the sign and therefore there are effectively only three data values), the refractive power (determined by C, S and θ) may be directly determined.

If the outputs of the phase difference measuring circuits 29 and 30 are made to correspond to the refractive power, the optical system will be able to be comparatively freely arranged without any complicated trouble.

Therefore, for example, a diaphragm 47 will be able to be moved to any position on the measuring optical axis.

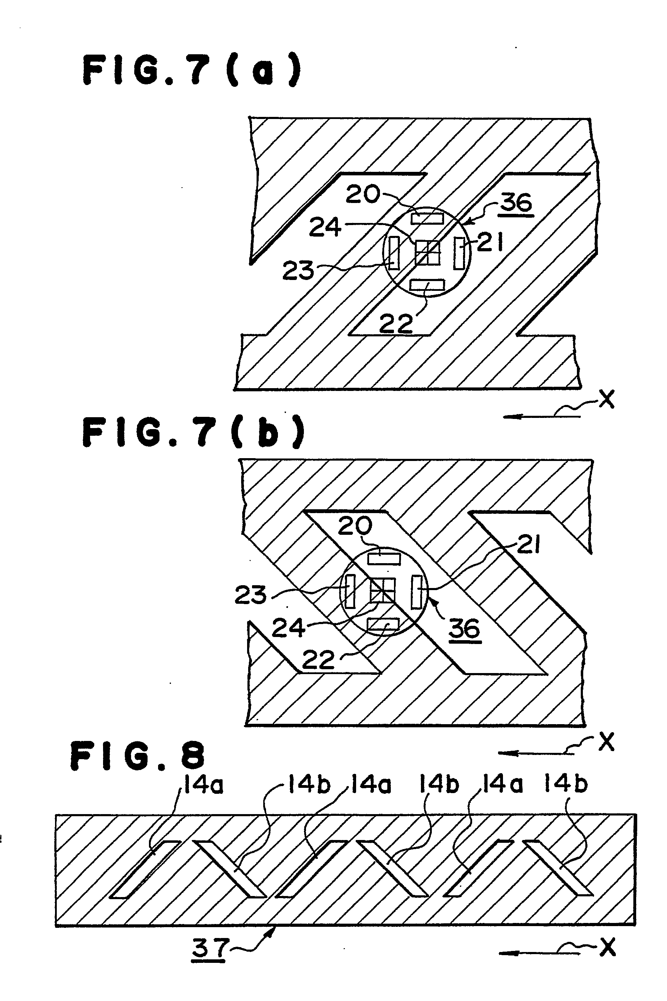

Further, if the direction of each pair of photoelectric converting elements is only known, it will not be required to be intersected rectangularly and will be able to be determined freely. For example, a photoelectric converter 36 in which the arrangement of the photoelectric converting elements 20, 21, 22 and 23 in the photoelectric converter 18 is rotated by 45 degrees may be used instead of the photoelectric converter 18. By the way, in this case, the views corresponding to the above described Figs. 6(a) and 6(b) are shown respectively in Figs. 7(a) and 7(b).

Further, as the angle of inclination of the slit-shaped illuminating beams scanning the eye being inspected may be set two or more different known angles, the slit-shaped apertures may be freely arranged. For example, a rotary chopper 37 in which the arrangement of the first slit-shaped apertures 14a and second slit-shaped apertures 14b is modified as shown in Fig. 8 may be used instead of the rotary chopper 13. Fig. 8 shows a developed view of the rotary chopper 37.

Further, as the angle of inclination of the slit-shaped illuminating beams scanning the eye being inspected may be set as two or more different known angles, if the angle of inclination of the slit-shaped apertures is only two or more different known angles, it will not be required to be limited to 45 degrees or the like but will be able to be freely determined. Also, the angle of inclination of the slit-shaped apertures need not be limited to be two different known angles but may be three or more different known angles. Also, the photoelectric converting elements need not be limited to be two pairs but may be three or more pairs.

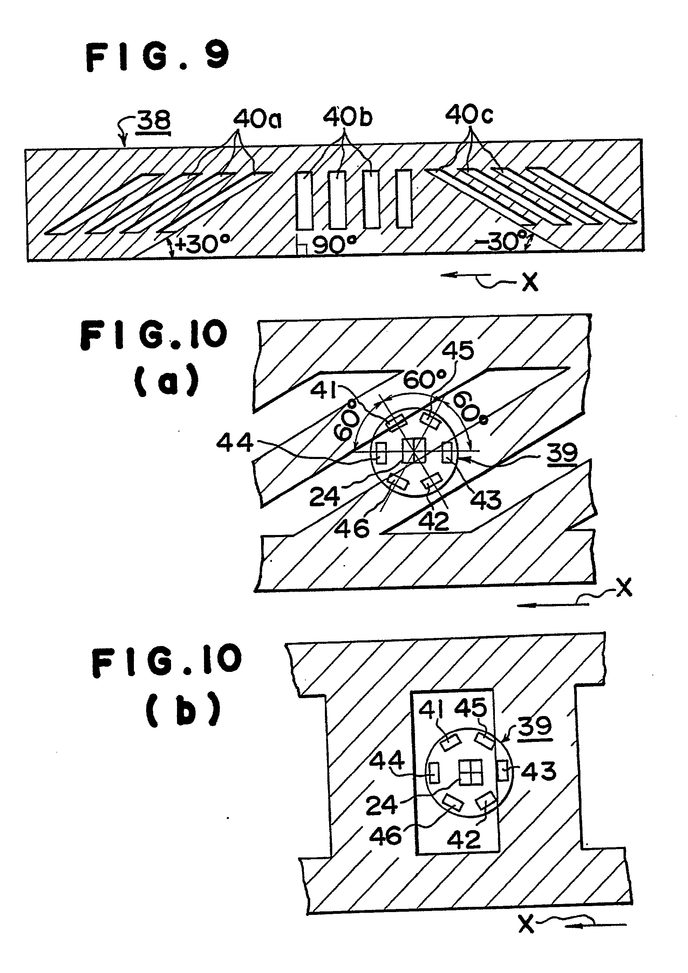

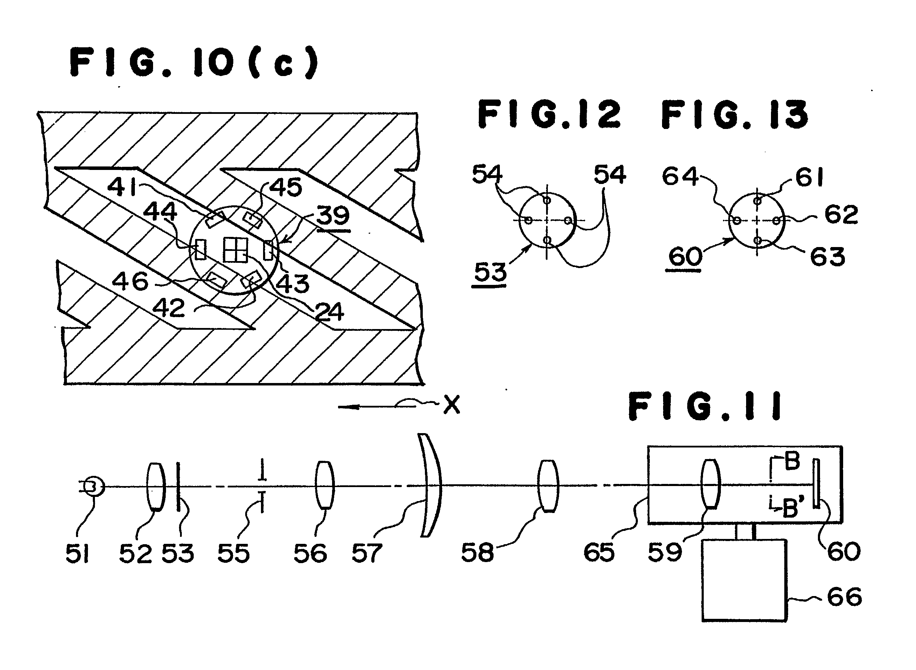

For example, a rotary chopper 38 as shown in Fig. 9 may be used instead of the rotary chopper 13 and a photoelectric converter 39 as shown in Figs. 10(a), 10(b) and 10(c) may be used instead of the photoelectric converter 18. Fig. 9 shows a developed view of the rotary chopper 38 in which the angle of inclination of the first slit-shaped apertures 40a to the rotating direction X is made +30 degrees, the angle of inclination of the second slit-shaped apertures 40b is made +90 degrees and the angle of inclination of the third slit-shaped apertures 40c is made -30 degrees. In a photoelectric converter 39, respective pairs of photoelectric converting elements 41 and 42, 43 and 44 and 45 and 46 are arranged symmetrically with respect to the optical axis on three lines respectively intersecting rectangularly with the optical axis and forming 60 degrees with one another.

In this case, if there is no astigmatism in the eye being inspected, when the slit-shaped illuminating beams based on the first slit-shaped apertures 40a are scanning the eye 7, the reflected light will run on the light receiving surface of the photoelectric converter 39 in the direction indicated by the arrow X as in Fig. 10(a), when the slit-shaped illuminating beams based on the second slit-shaped apertures 40b are scanning the eye 7, the reflected light will run on the light receiving surface of the photoelectric converter 39 in the direction indicated by the arrow X as in Fig. 10(b) and, when the slit-shaped illuminating beams based on the third slit-shaped apertures 40c are scanning the eye 7, the reflected light will run on the light receiving surface of the photoelectric converter 39 in the direction indicated by the arrow X as in Fig. 10(c).

Therefore, if when the slit-shaped illuminating beams based on the first slit-shaped apertures 40a scan the eye 7, the value obtained from the phase difference of the output signals of the photoelectric converting elements 41 and 42 is represented by $D_1''$, and when the slit-shaped illuminating beams based on the second slit-shaped apertures 40b scan the eye 7, the value obtained from the phase difference of the output signals of the photoelectric converters 43 and 44 is represented by $D_2''$ and, when the slit-shaped illuminating beams based on the third slit-shaped apertures 40c scan the eye 7, the value obtained from the phase difference of the output signals of the photoelectric converters 45 and 46 is represented by $D_3''$, then

$$D_1'' = S + C \cos^2 (\theta - 60) \qquad (9)$$

$$D_2'' = S + C \cos^2 \theta \qquad (10)$$

$$D_3'' = S + C \cos^2 (\theta + 60) \qquad (11)$$

Therefore, as the operating circuit 9 may be arranged to determine the three unknowns (C, S and $\theta$) from the three measured data values ($D_1''$, $D_2''$ and $D_3''$) using equations (9), (10) and (11) whereby the cylinder axis angle $\theta$, the spherical refractive power S and the cylindrical refractive power C may be determined.

By the way, in this case also, a waveform shaping circuit and phase difference measuring circuit will be provided.

In the above explanation, the shapes of the slit-shaped apertures shown in Figs. 5, 8 and 9 have been explained by using the developed views of the rotary choppers. However, when the chopper is actually cylindrical, the slit-shaped apertures will preferably have a fixed angle of inclination.

In the above explanation, the eye refractive power measuring apparatus has been described. However, the above described apparatus can be used also as a lens meter without any modification in the operating principle. In the case of measuring the eye refractive power, it is a feature in the formation that, in order to measure the beams reflected in the eye bottom, the illuminating beams pass twice through the inspected optical system (the crystal body of the inspected eye 7) and the projecting light path and measuring light path are used partly in common. Usually, in the case of a lens meter, the lens to be inspected can be measured by one pass and the projecting light path and measuring light path are formed to hold the inspected lens. Needless to say, if the beams passing through the inspected lens are again made incident upon the inspected lens by a reflector, the formation will be able to be made the same as of the above described eye refractive power measuring apparatus.

An embodiment of the apparatus of the present invention in the form of a lens meter will now be briefly explained. By the way, this embodiment relates to an improvement of the lens meter disclosed in Japanese Unexamined Patent Application Publication No. 168137/1982.

Fig. 11 is a schematic view of an apparatus of the invention the form of optical lens meter.

The reference numeral 51 represents a light source, 52 represents a condenser lens, 53 represents a diaphragm having four apertures 54 (the shape as seen in the optical axis direction being shown in Fig. 12) arranged on a circle concentric with the optical axis as intersecting rectangularly with the circle, 55 represents a diaphragm (as 47 in Fig. 1) and 56 represents a collimator lens, the diaphragm 55 preferably being substantially at the focus of the collimator lens 56. The reference numeral 57 represents a lens to be inspected. The four apertures 54 of the diaphragm 53 form images on the inside surface of the lens 57. The reference numerals 58 and 59 represent collimator lenses and 60 represents a photoelectric converter in which, as shown in Fig. 13, photoelectric converting elements 61, 62, 63 and 64 are arranged as a circle concentric with the optical axis as intersecting rectangularly with the circle. The rectangularly intersecting axes must coincide respectively with the rectangularly intersecting axes of the four apertures 54 of the diaphragm 53. Also, the photoelectric converter 60 must be substantially conjugated with the inside surface of the inspected lens 57. The reference numeral 65 represents a rotary chopper and 66 represents a motor for rotating the rotary chopper 65.

In the optical system arranged as described above, the beams emitted from the light source 51 will pass through the condenser lens 52, diaphragms 53 and 55 and collimator lens 56, will be refracted by the inspected lens 57, will be chopped by the rotary chopper 65, will pass through the collimator lens 59 and will reach the photoelectric converter 60. Therefore, phase differences proportional to the refractive power of the inspected lens 57 will be produced respectively between the output signals of the photoelectric converting elements 61 and 63 and between the output signals of the photoelectric converting elements 62 and 64 of the photoelectric converter 60.

Here, if the shape of the rotary chopper 65 is made the same as of the rotary chopper 13 shown in Fig. 5 or the rotary chopper 38 shown in Fig. 9, the refractive power of the inspected lens 57 will be able to be determined using equations (5) to (8) or (9) to (11).

The operating and other circuit used in this embodiment of the apparatus of the invention may of course be analogous to those described in connection with the earlier embodiments, the automatic eye refractive power measuring apparatus.

Further, an embodiment of the apparatus of the present invention in the form of a radius of curvature measuring apparatus will now be explained. This embodiment relates to an improvement of the automatic radius of curvature measuring apparatus described in Japanese Unexamined Patent Application Publication No. 197405/1982.

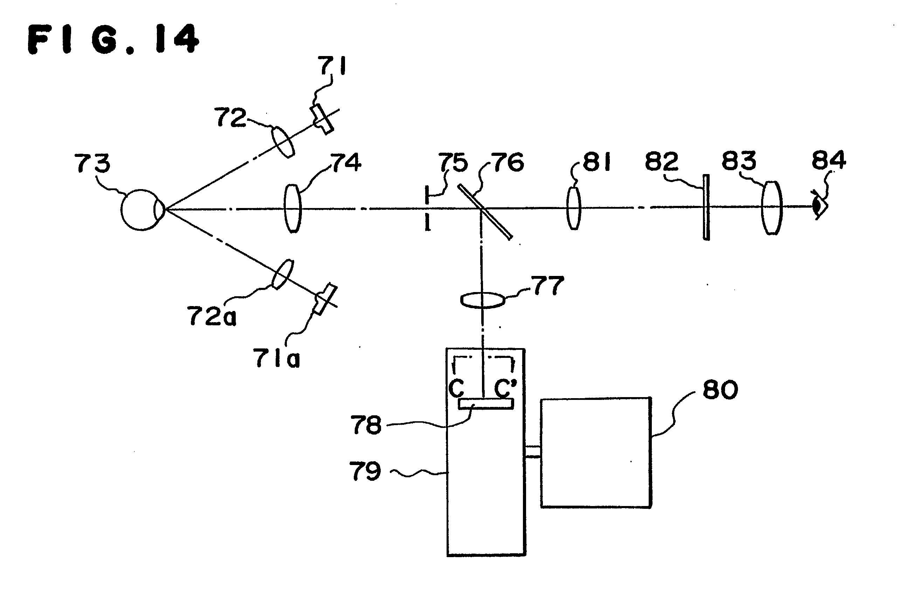

Fig. 14 is a schematic view of an apparatus according to the invention in the form of a radius of curvature measuring apparatus.

The reference numerals 71 and 71a represent light sources and 72 and 72a represent collimator lenses. Another pair of light sources and another pair of collimator lenses not illustrated are located in the direction vertical to the paper surface of Fig. 14. These two pairs of beams correspond to the four apertures 54 of the diaphragm 53 shown in Fig. 11. The reference numeral 73 represents an eye to be inspected and 74 represents a collimator lens. It is preferable that the focus of the collimator lens 74 substantially coincides with the cornea of the inspected eye 73. The reference numeral 75 represents a diaphragm which is located preferably at the focus of the collimator lens 74. The reference numberal 76 represents a beam splitter, 77, 78, 79 and 80 are components of a measuring optical system and 81, 82, 83 and 84 are components of a positioning optical system, 77 and 81 representing collimator lenses by which images projected on the cornea of the inspected eye are formed respectively on a photoelectric converter 78 and a reticle 82. It is preferable that the foci of the collimator lenses 77 and 81 substantially coincide with the diaphragm 75. The reference numeral 79 represents a rotary chopper and 80 represents a motor for rotating the rotary chopper. A four-divided element is used as shown in Fig. 15 for the photoelectric converter 78. By the way, such a four-divided element as is shown in Fig. 15 may be used also for the photoelectric converter 60 in the optical system of the lens meter shown in Fig. 11. The reference number 83 represents an eyepiece and 84 represents an inspector's eye.

In the apparatus described above, two pairs of four beams emitted from the light sources 71 and 71a and the light sources intersecting rectangularly with these light sources will be reflected by the cornea of the inspected eye 73, will pass through the collimator lens 74 and diaphragm 75 and will be split by the beam splitter 76.

Part of the split beams will pass through the collimator lens 81, will further pass through the recticle 82 and eyepiece 83 and will be observed by the inspector's eye. Here, the inspector will focus and center the four beams projected on the cornea of the inspected eye 73.

The reflected portion of the split beams will pass through the collimator lens 77 and will reach the photoelectric converter 78. Phase differences proportional to the respective radii of curvature will be pro-

EP 0 210 722 B1

duced between the output signals of the respective pairs of the four-divided element of the photoelectric converter 78.

Here, if the shape of the rotary chopper 79 is made the same as of the rotary chopper 13 shown in Fig. 5 or the rotary chopper 38 shown in Fig. 9, the radius of curvature of the inspected eye 73 will be able to be determined using equations (5) to (8) or (9) to (11).

The operating and other circuits used in this embodiment of the apparatus of the invention may of course to analogous to the circuits used in the first described embodiment, the automatic eye refractive power measuring apparatus.

Also, it is apparent that, if a lens is arranged instead of the inspected eye 73, it will of course be possible to determine the radius of curvature of the lens.

As detailed in the above, according to the present invention, the conventionally required prism is no longer required, a single light source may be used, the use of an image rotating prism is not required and therefore manufacture is simplified, measuring time is reduced and the refractive power and radius of curvature of an optical system under investigation can be measured with high precision.

## Claims

1. Apparatus for measuring the refractive power of an optical system or the radius of curvature of a lens of an optical system, said apparatus comprising:

means (11, 6) for emitting light to impinge on said optical system (7); means (17, 47) for directing said light from said optical system (7) onto at least two pairs (20, 22 and 21, 23) of photoelectric converting elements;

means (13, 15) for scanning said light over said optical system (7) and/or said converting elements, said means defining beams of elongate cross-section; and

determining means (9) for determining said refractive power or radius of curvature on the basis of the phase differences between the output signals of said pairs of converting elements,

characterized in that said means for scanning is arranged to define said beams with the elongate cross-sections thereof inclined at at least two angles of inclination relative to the direction of scanning (X), and in that said apparatus comprises means (19) for providing to said determining means (9) a signal identifying said angle of inclination of the light beam scanning said optical system and/or said converting elements.

2. An apparatus as claimed in claim 1 for measuring the radius of curvature of the lens of an optical system (73, Fig. 14) wherein the beam from four light sources (71, 71a) is projected onto said lens by four collimating lenses (72, 72a) and said means (79, 80) for scanning is arranged to define beams of light reflected from said lens of said optical system (73) and to scan said photoelectric converting elements (78) therewith.

3. An apparatus as claimed in claim 1, for measuring the refractive power of an optical system (57, Fig. 11) wherein said means (65, 59, 66) for scanning is arranged to define beams of scanning light transmitted through said optical system (57) and said photoelectric converting elements (60) are arranged in the focal plane of a collimator lens (59).

4. Apparatus as claimed in any one of claims 1 to 3 wherein said means (13, 37, Figs. 5, 8) for scanning is so arranged as to define light beams whose elongate cross-sections are inclined at +45° and −45° to the direction of scanning (X).

5. An apparatus as claimed in any one of claims 1 to 3 wherein said means (38, 40, Fig. 9) for scanning is so arranged as to define light beams whose elongate cross-sections are inclined at +30°, +90° and −30° to said direction of scanning (X).

## Patentansprüche

1. Vorrichtung zur Messung der Brechkraft eines optischen Systems oder des Krümmungsradius einer Linse eines optischen Systems, wobei die Vorrichtung aufweist:

eine Einrichtung (11, 6) zur Aussendung von Licht zum Einfall auf das optische System (7),

eine Einrichtung (17, 47) zur Ausrichtung des Lichts von dem optischen System (7) auf mindestens zwei Paare (20, 22 und 21, 23) von photoelektrischen Wandlerelementen,

eine Einrichtung (13, 15) für eine Abtastbewegung des Lichtes über das optische System (7) und/oder die Wandlerelemente, wobei die Einrichtung Strahlen von länglichem Querschnitt festlegt, und

eine Bestimmungseinrichtung (9) zur Bestimmung der Brechkraft oder des Krümmungsradius auf der Grundlage der Phasenunterschiede zwischen den Ausgangssignalen der Paare von Wandlerelementen,

dadurch gekennzeichnet, daß die Abtasteinrichtung dazu beschaffen ist, die Strahlen mit ihren länglichen Querschnitten unter mindestens zwei Neigungswinkeln in bezug auf die Abtastrichtung (X) geneigt festzulegen, und daß die Vorrichtung eine Einrichtung (19) zur Lieferung eines den Neigungswinkel des das optische System und/oder die Wandlerelemente abtastenden Lichtstrahls identifizierenden Signals an die Bestimmungseinrichtung (9) aufweist.

9

2. Vorrichtung nach Anspruch 1 zur Messung des Krümmungsradius der Linse eines optischen Systems (73, Fig. 14), bei der der Strahl aus vier Lichtquellen (71, 71a) durch vier Kollimatorlinsen (72, 72a) auf die Linse projiziert wird und die Abtasteinrichtung (79, 80) dazu beschaffen ist, von der Linse des optischen Systems (73) reflektierte Strahlen festzulegen und damit die photoelektrischen Wandlerelemente (78) abzutasten.

3. Vorrichtung nach Anspruch 1 zur Messung der Brechkraft eines optischen Systems (57, Fig. 11), bei der die Abtasteinrichtung (65, 59, 66) dazu beschaffen ist, durch das optische System (57) hindurch übertragene Abtastlichtstrahlen festzulegen, und die photoelektrischen Wandlerelemente (60) in der Brennebene einer Kollimatorlinse (59) angeordnet sind.

4. Vorrichtung nach einem der Ansprüche 1 bis 3, bei der die Abtasteinrichtung (13, 37, Figuren 5, 8) derart beschaffen ist, daß sie Lichtstrahlen festlegt, deren längliche Querschnitte +45° und –45° gegenüber der Abtastrichtung (X) geneigt sind.

5. Vorrichtung nach einem der Ansprüche 1 bis 3, bei der die Abtasteinrichtung (38, 40, Fig. 9) derart beschaffen ist, daß sie Lichtstrahlen festlegt, deren längliche Querschnitte +30°, +90° und –30° gegenüber der Abtastrichtung (X) geneigt sind.

## Revendications

1. Dispositif de mesure du pouvoir de réfraction d'un système optique ou du rayon de courbure d'une lentille d'un système optique, ce dispositif comprenant:
   – des moyens (11, 6) permettant d'émettre de la lumière tombant sur le système optique (7);
   – des moyens (17, 47) permettant de diriger la lumière provenant du système optique (7) sur au moins deux paires (20, 22 et 21, 23) d'éléments de conversion photo-électriques;
   – des moyens (13, 15) permettant de balayer la lumière sur le système optique (7) et/ou sur les éléments de conversion, ces moyens définissant des faisceaux de section allongée; et
   – des moyens de détermination (9) permettant de déterminer le pouvoir de réfraction ou le rayon de courbure à partir des différences de phase entre les signaux de sortie des paires d'éléments de conversion, dispositif caractérisé en ce que les moyens de balayage sont conçus de manière à donner aux faisceaux des sections transversales allongées inclinées suivant au moins deux angles d'inclinaison par rapport à la direction de balayage (X) et en ce que l'appareil comprend des moyens (19) permettant de fournir aux moyens de détermination (9) un signal identifiant l'angle d'inclinaison du faisceau de lumière balayant le système optique et/ou les éléments de conversion.

2. Dispositif selon la revendication 1, pour mesurer le rayon de courbure de la lentille d'un système optique (73, figure 14), dispositif caractérisé en ce que le faisceau provenant de quatre sources de lumière (71, 71a) est projeté sur la lentille de collimateur (72, 72a), et en ce que les moyens (79, 80) de balayage sont conçus pour définir des faisceaux de lumière réfléchis par la lentille du système optique (73), et pour balayer les éléments de conversion photo-électriques (78) au moyen de ces faisceaux.

3. Dispositif selon la revendication 1, pour mesurer le pouvoir de réfraction d'un système optique (57, figure 11), dispositif caractérisé en ce que les moyens (65, 59, 66) de balayage sont conçus pour définir des faisceaux de lumière de balayage transmis à travers le système optique (57), et en ce que les éléments de conversion photo-électriques (60) sont disposés dans le plan focal d'une lentille de collimateur (59).

4. Dispositif selon l'une quelconque des revendications 1 à 3, caractérisé en ce que les moyens (13, 37, figures 5, 8) de balayage sont conçus de manière à définir les faisceaux de lumière dont les sections transversales allongées sont inclinées de +45 degrés et –45 degrés par rapport à la direction de balayage (X).

5. Dispositif selon l'une quelconque des revendications 1 à 3, caractérisé en ce que les moyens (38, 40, figure 9) de balayage sont conçus de manière à définir des faisceaux de lumière dont les sections transversales allongées sont inclinées de +30 degrés, +90 degrés et –30 degrés par rapport à la direction de balayage (X).

# FIG. 1

# FIG. 2

# FIG. 3

# FIG.4

# FIG.5

# FIG. 6 ( a )

# FIG. 6 ( b )

# FIG. 7 (a)

# FIG. 7 (b)

# FIG. 8

# FIG. 9

38  40a  40b  40c
+30°  90°  −30°
X

# FIG. 10 (a)

45
41  60°
60°  60°
44
24  39
46  43
42
X

# FIG. 10 (b)

41 45
44  39
24  43
46  42
X

FIG. IO(c)

FIG.12 FIG.13

FIG.II

# FIG. 14

EP 0 210 722 B1

# F I G . 15

# F I G . 16

# FIG. 17 (a)

# FIG. 17 (b)